# EUROPÄISCHE PATENTSCHRIFT

(11) **EP 1 173 425 B1**
(45) Veröffentlichungstag und Bekanntmachung des Hinweises auf die Patenterteilung: **20.07.2005**
(21) Anmeldenummer: 00926822.8
(22) Anmeldetag: 06.04.2000
(51) Int. Cl.: C07D 249/12, A01N 43/653

(54) **(-)-ENANTIOMERES DES 2-[2-(1-CHLOR-CYCLOPROPYL)-3-(2-CHLORPHENYL) -2-HYDROXYPROPYL]-2,4-DIHYDRO-[1,2,4]-TRIAZOL-3-THIONS**
(-)-ENANTIOMER OF THE 2- 2-(1- CHLOROCYCLOPROPYL)-3- (2-CHLOROPHENYL) -2- HYDROXYPROPYL] -2,4- DIHYDRO- 1,2,4] -TRIAZOLE -3-THIONE
ENANTIOMERE(-) DE LA 2- 2-(1-CHLOROCYCLOPROPYL)-3-(2-CHLOROPHENYL)-2-HYDROXYPROPYL]-2,4-DIHYDRO 1,2,4]TRIAZOL-3-THIONE

(30) Priorität: 19.04.1999 DE 19917617
(43) Veröffentlichungstag der Anmeldung: 23.01.2002
(73) Patentinhaber: Bayer CropScience AG, 40789 Monheim (DE)
(72) Erfinder: GROSSER, Rolf, D-51373 Leverkusen (DE); JAUTELAT, Manfred, D-51399 Burscheid (DE); MAULER-MACHNIK, Astrid, D-42799 Leichlingen (DE); DUTZMANN, Stefan, D-40764 Langenfeld (DE); HÄNSSLER, Gerd, D-51381 Leverkusen (DE); STENZEL, Klaus, D-40595 Düsseldorf (DE)
(86) Internationale Anmeldenummer: PCT/EP2000/003066
(87) Internationale Veröffentlichungsnummer: WO 2000/063188

(56) Entgegenhaltungen:
- WO-A-96/16048
- DE-A- 19 744 400
- DE-A- 19 744 401
- DE-A- 19 744 706
- DE-A- 19 839 688

## Beschreibung

Die vorliegende Erfindung betrifft das neue (-)-Enantiomere des 2-[2-(1-Chlor-cyclopropyl)-3-(2-chlorphenyl)-2-hydroxypropyl]-2,4-dihydro-[1,2,4]-triazol-3-thions, ein Verfahren zu dessen Herstellung und dessen Verwendung als Mikrobizid.

Es ist bereits bekannt, dass das Racemat des 2-[2-(1-Chlor-cyclopropyl)-3-(2-chlorphenyl)-2-hydroxypropyl]-2,4-dihydro-[1,2,4]-triazol-3-thions fungizide Eigenschaften besitzt (vgl. WO 96-16 048). Die Wirksamkeit dieser Substanz ist gut, lässt aber bei sehr niedrigen Aufwandmengen in manchen Fällen zu wünschen übrig.

Es wurde nun das neue (-)-Enantiomere des 2-[2-(1-chlor-cyclopropyl)-3-(2-chlorphenyl)-2-hydroxypropyl]-2,4-dihydro-[1,2,4]-triazol-3-thions der Formel 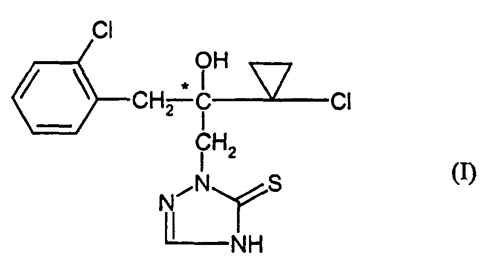 gefunden.

Unter dem (-)-Enantiomeren ist hier jeweils dasjenige Enantiomere zu verstehen, das die Schwingungsebene von linear polarisiertem Licht der Natrium-D-Linie nach links dreht.

Weiterhin wurde gefunden, dass man das (-)-Enantiomere des 2-[2-(1-Chlor-cyclopropyl)-3-(2-chlor-phenyl)-2-hydroxypropyl]-2,4-dihydro-[1,2,4]-triazol-3-thions der Formel (I) erhält, wenn man
a) racemisches 2-[2-(1-Chlor-cyclopropyl)-3-(2-chlor-phenyl)-2-hydroxypropyl]-2,4-dihydro-[1,2,4]-triazol-3-thion der Formel 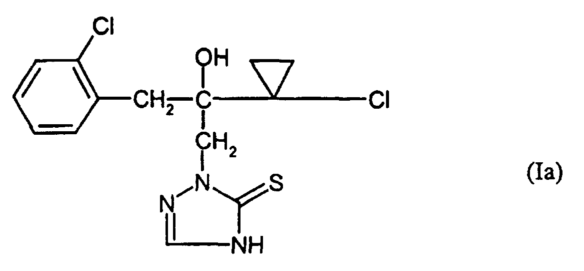 an einer chiralen stationären Kieselgelphase basierend auf dem optisch aktiven Monomer N-Methacryloyl-L-leucin-3-(2,4-dimethylpentyl)-amid mit Ethylacetat als Elutionsmittel bei Temperaturen zwischen 20°C und 25°C chromatographiert,
b) das Eluat unter vermindertem Druck einengt und
c) das dabei anfallende Produkt aus Toluol umkristallisiert.

Schließlich wurde gefunden, dass das neue (-)-Enantiomere des 2-[2-(1-Chlor-cyclopropyl)-3-(2-chlor-phenyl)-2-hydroxypropyl]-2,4-dihydro-[1,2,4]-triazol-3-thions der Formel (I) sehr gute mikrobizide Eigenschaften besitzt und sich sowohl im Pflanzenschutz als auch im Materialschutz zur Bekämpfung unerwünschter Mikroorganismen, wie Fungi, verwenden lässt.

Überraschenderweise zeigt das erfindungsgemäße (-)-Enantiomere des 2-[2-(1-Chlorcyclopropyl)-3-(2-chlorphenyl)-2-hydroxypropyl]-2,4-dihydro-[1,2,4]-triazol-3-thions der Formel (I) eine wesentlich bessere fungizide Wirksamkeit als das entsprechende (+)-Enantiomere und das entsprechende Racemat, das als hoch wirksamer Wirkstoff mit fungiziden Eigenschaften bekannt ist.

Das erfindungsgemäße (-)-Enantiomere kann ganz oder teilweise in der "Thiono"-Form der Formel 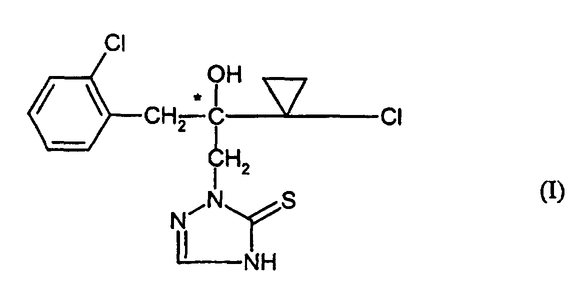 oder in der tautomeren "Mercapto"-Form der Formel 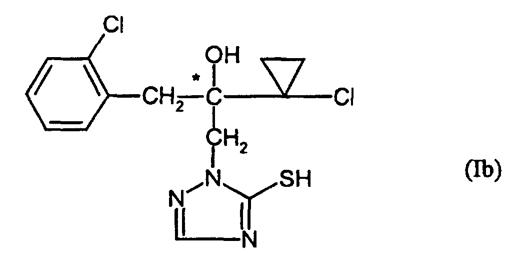 vorliegen. Der Einfachheit halber wird jeweils nur die "Thiono"-Form aufgeführt.

In den Formeln (I) und (Ib) ist das asymmetrisch substituierte Kohlenstoffatom jeweils durch ein (*) gekennzeichnet.

Das racemische 2-[2-(1-Chlor-cyclopropyl)-3-(2-chlor-phenyl)-2-hydroxypropyl]-2,4-dihydro-[1,2,4]-triazol-3-thion der Formel (Ia), das bei der Durchführung des erfindungsgemäßen Verfahrens als Ausgangsmaterial benötigt wird, ist bekannt (vgl. WO 96-16 048).

Bei der Durchführung des erfindungsgemäßen Verfahrens arbeitet man nach Methoden der präparativen Chromatographie, vorzugsweise nach der Methode der High Performance Liquid Chromatography (=HPLC). Das dabei eingesetzte Trennmaterial ist bekannt (vgl. EP-A 0 397 917).

Der Substanzgehalt im Eluat wird durch photometrische Detektion ermittelt. Die aufgefangenen Eluat-Fraktionen werden analytisch auf Enantiomerenreinheit hin untersucht. Fraktionen, die jeweils das gleiche Enantiomere enthalten, werden zusammengefasst und unter vermindertem Druck eingeengt. Anschließend wird das erhaltene Produkt aus Toluol umkristallisiert.

Das zuerst eluierte Produkt ist das erfindungsgemäße (-)-Enantiomere. Aus weiteren, später abfließenden Fraktionen lässt sich das entsprechende (+)-Enantiomere isolieren.

Der erfindungsgemäße Wirkstoff weist eine starke mikrobizide Wirkung auf und kann zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit des Wirkstoffes in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lässt sich der erfindungsgemäße Wirkstoff mit besonders gutem Erfolg zur Bekämpfung von Krankheiten im Wein-, Obst- und Gemüseanbau einsetzen, wie beispielsweise gegen echte Mehltaupilze, wie Sphaerotheca, Uncinula, gegen Erysiphe-Arten sowie Blattflecken, wie Venturia und Alternaria-Arten. Mit gutem Erfolg werden auch Getreidekrankheiten, wie Erysiphe-, Leptosphaeria- oder Pyrenophora-Arten, und Reiskrankheiten, wie Pyricularia-Arten, bekämpft.

Der erfindungsgemäße Wirkstoff eignet sich auch zur Steigerung des Ernteertrages. Er ist außerdem mindertoxisch und weist eine gute Pflanzenverträglichkeit auf.

Im Materialschutz lässt sich der erfindungsgemäße Wirkstoff zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Der Wirkstoff kann in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann als solcher oder in seinen Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacril-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Edifenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB), Quinoxyfen,
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetruolo[1,5-a]quinuolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2,dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin-Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaphorthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
   Elfusilanate, Emamectin, Empenthrin, Endosulfan, Entomopfthora spp., Esfenvalerate, Ethiofencarb, Ethion, Ethoprophos, Etofenprox, Etoxazole, Etrimphos,
   Fenamiphos, Fenazaquin, Fenbutatinoxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
   Granuloseviren,
   Halofenozide, HCH, Heptenophos, Hexaflumuron, Hexythiazox, Hydroprene,
   Imidacloprid, Isazophos, Isofenphos, Isoxathion, Ivermectin,
   Kempolyederviren,
   Lamda-cyhalothrin, Lufenuron,
   Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
   Naled, Nitenpyram, Nithiazine, Novaluron,
   Omethoat, Oxamyl, Oxydemethon M,
   Paecilomyces fumosoroseus, Parathion A, Parathion M, Permethrin, Phenthoat, Phorat, Phosalon, Phosmet, Phosphamidon, Phoxim, Pirimicarb, Pirimiphos A, Pirimiphos M, Profenophos, Promecarb, Propoxur, Prothiophos, Prothoat, Pymetrozine, Pyraclofos, Pyresmethrin, Pyrethrum, Pyridaben, Pyridathion, Pyrimidifen, Pyriproxifen,
   Quinalphos,
   Ribavirin,
   Salithion, Sebufos, Silafluofen, Spinosad, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-Cypermethrin, Zolaprofos

   (1R-cis)-[5-(Phenylmethyl)-3-furanyl]-methyl-3-[(dihydro-2-oxo-3(2H)-furanyliden)-methyl]-2,2-dimethylcyclopropancarboxylat,
   (3-Phenoxyphenyl)-methyl-2,2,3,3-tetramethylcyclopropanecarboxylat,
   1-[(2-Chlor-5-thiazolyl)methyl]tetrahydro-3,5-dimethyl-N-nitro-1,3,5-triazin-2(1H)-imin,
   2-(2-Chlor-6-fluorphenyl)-4-[4-(1,1-dimethylethyl)phenyl]-4,5-dihydro-oxazol,
   2-(Acetyloxy)-3-docecyl-1,4-naphthalindion,
   2-Chlor-N-[[[4-(1-phenylethoxy)-phenyl]-amino]-carbonyl]-benzamid,
   2-Chlor-N-[[[4-(2,2-dichlor-1,1-difluorethoxy)-phenyl]-amino]-carbonyl]-benzamid,
   3-Methylphenyl-propylcarbamat,
   4-[4-(4-Ethoxyphenyl)-4-methylpentyl]-1-fluor-2-phenoxy-benzol,
   4-Chlor-2-(1,1-dimethylethyl)-5-[[2-(2,6-dimethyl-4-phenoxyphenoxy)ethyl]thio]-3(2H)-pyridazinon,
   4-Chlor-2-(2-chlor-2-methylpropyl)-5-[(6-iod-3-pyridinyl)methoxy]-3(2H)-pyridazinon,
   4-Chlor-5[(6-chlor-3-pyridinyl)methoxy]-2-(3,4-dichlorphenyl)-3(2H)-pyridazinon,
   Bacillus thuringiensis strain EG-2348,
   Benzoesäure (2-benzoyl-1-(1,1-dimethylethyl)-hydrazid,
   Butan 2,2-dimethyl-3-(2,4-dichlorphenyl)-2-oxo-1-oxaspiro[4,5]dec-3-en-4-yl-ester,
   Dihydro-2-(nitromethylen)-2H-1,3-thiazine-3(4H)-carboxaldehyd,
   Ethyl-[2-[[1,6-dihydro-6-oxo-1-(phenylmethyl)-4-pyridazinyl]oxy]ethyl]-carbamat,
   N-(3,4,4-Trifluor-1-oxo-3-butenyl)-glycin,
   N-(4-Chlorphenyl)-3-[4-Difluormethoxy)phenyl]-4,5-dihydro-4-phenyl-1H-pyrazol-1-carboxamid,
   N-[(2-Chlor-5-thiazolyl)methyl]-N'-methyl-N"-nitro-guanidin,
   N-Methyl-N'-(1-methyl-2-propenyl)-1,2-hydrazindicarbothioamid,
   N-Methyl-N'-2-propenyl-1,2-hydrazindicarbothioamid,
   O,O-Diethyl-[2-(dipropylamino)-2-oxoethyl]-ethylphosphoramidothioat.

Auch eine Mischung mit anderen bekannten Wirkstoffen, wie Herbiziden oder mit Düngemitteln und Wachstumsregulatoren ist möglich.

Der Wirkstoff kann als solcher, in Form seiner Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz des erfindungsgemäßen Wirkstoffes als Fungizid kann die Aufwandmenge je nach Applikationsart innerhalb eines größeren Bereiches variiert werden. Bei der Behandlung von Pflanzenteilen liegen die Aufwandmengen an Wirkstoff im allgemeinen zwischen 0,1 und 10 000 g/ha, vorzugsweise zwischen 10 und 1 000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10 000 g/ha, vorzugsweise zwischen 1 und 5 000 g/ha.

Die zum Schutz technischer Materialien verwendeten Mittel enthalten den Wirkstoff im allgemeinen in einer Menge von 1 bis 95 %, bevorzugt von 10 bis 75 %.

Die Anwendungskonzentrationen des erfindungsgemäßen Wirkstoffes richten sich nach der Art und dem Vorkommen der zu bekämpfenden Mikroorganismen sowie nach der Zusammensetzung des zu schützenden Materials. Die optimale Einsatzrnenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-% bezogen auf das zu schützende Material.

Die Wirksamkeit und das Wirkungsspektrum des erfindungsgemäß im Materialschutz zu verwendenden Wirkstoffes bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäße Verbindung.

Die Herstellung und die Verwendung des erfindungsgemäßen Wirkstoffes geht aus den folgenden Beispielen hervor.

### Herstellungsbeispiel

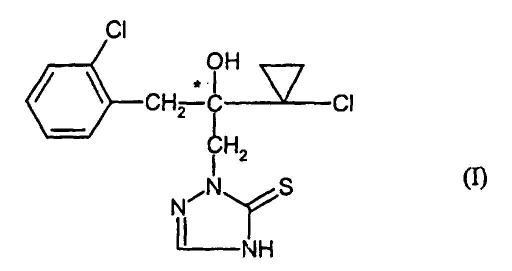

300 g racemisches 2-[2-(1-Chlor-cyclopropyl)-3-(2-chlor-phenyl)-2-hydroxypropyl]-2,4-dihydro-[1,2,4]-triazol-3-thion werden in Portionen von 0,2 g an einer chiralen stationären Kieselgelphase (CSP) basierend auf dem optisch aktiven Monomer N-Methacryloyl-L-leucin-3-(2,4-dimethyl-pentyl)-amid mit Ethylacetat als Elutionsmittel bei Raumtemperatur (etwa 23°C) nach der HPLC-Methode getrennt. Dabei wird eine photometrische Detektion des Eluates vorgenommen. Im einzelnen erfolgt die präparative Trennung unter folgenden Bedingungen.

| | |
|---|---|
| Säule | CSP wie oben, 10 µm; 570 * 50 mm ID |
| Eluens | Ethylacetat |
| Fluss | 100 ml/min |
| Detektion | UV; 254 nm |
| Probenaufgabe | 0,2 g gelöst in 20 ml Ethylacetat |

Diejenigen Fraktionen, die jeweils das gleiche Enantiomere enthalten, werden zusammengefasst und unter vermindertem Druck eingeengt. Das anfallende Produkt wird anschließend aus Toluol umkristallisiert.

Unter den angegebenen Bedingungen eluiert zuerst das linksdrehende Enantiomere und danach das rechtsdrehende Enantiomere.

Man erhält auf diese Weise 117 g an dem (-)-Enantiomeren des 2-[2-(1-Chlor-cyclopropyl)-3-(2-chlor-phenyl)-2-hydroxypropyl]-2,4-dihydro-[1,2,4]-triazol-3-thions in Form einer Festsubstanz vom Schmelzpunkt 123 bis 124°C.

Spezifischer Drehwert:
[α]²⁰_{D} =-55,5° (10 mg / 1 ml Chloroform)

In analoger Weise erhält man 119 g an dem (+)-Enantiomeren in Form einer Festsubstanz vom Schmelzpunkt 122-123°C.

Spezifischer Drehwert:
[α]²⁰_{D} = -54,9° (10 mg / 1 ml Chloroform)

### Verwendungsbeispiele

### Beispiel A

### Cochliobolus sativus-Test (Gerste) / protektiv

| | | |
|---|---|---|
| Lösungsmittel | 10 | Gewichtsteile N-Methyl-pyrrolidon |
| Emulgator | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung in der angegebenen Aufwandmenge. Nach dem Antrocknen des Spritzbelages werden die Pflanzen mit einer Konidiensuspension von *Cochliobolus sativus* besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von 20°C und einer relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

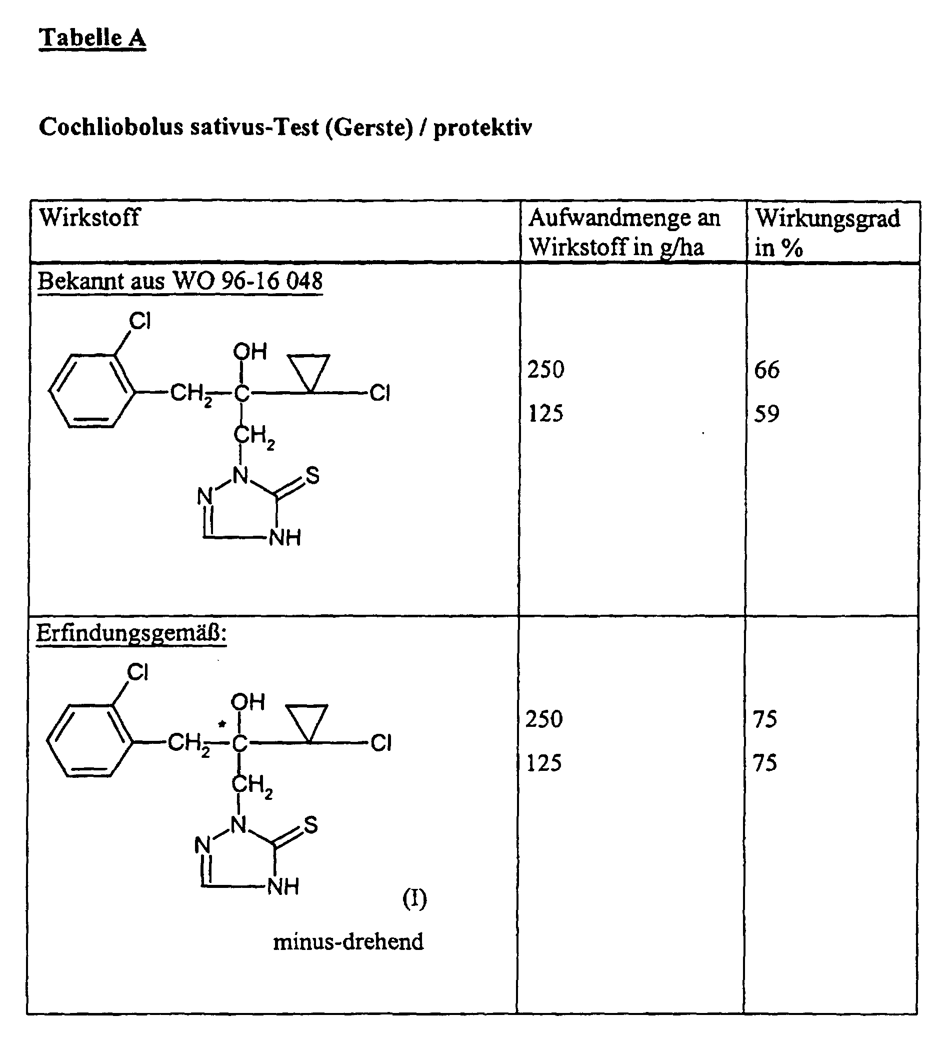

### Beispiel B

### Fodosphaera-Test (Apfel) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung in der angegebenen Aufwandmenge. Nach Antrocknen des Spritzbelages werden die Pflanzen mit einer wässrigen Sporensuspension des Apfelinehltauerregers *Podosphaera leucotricha* inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

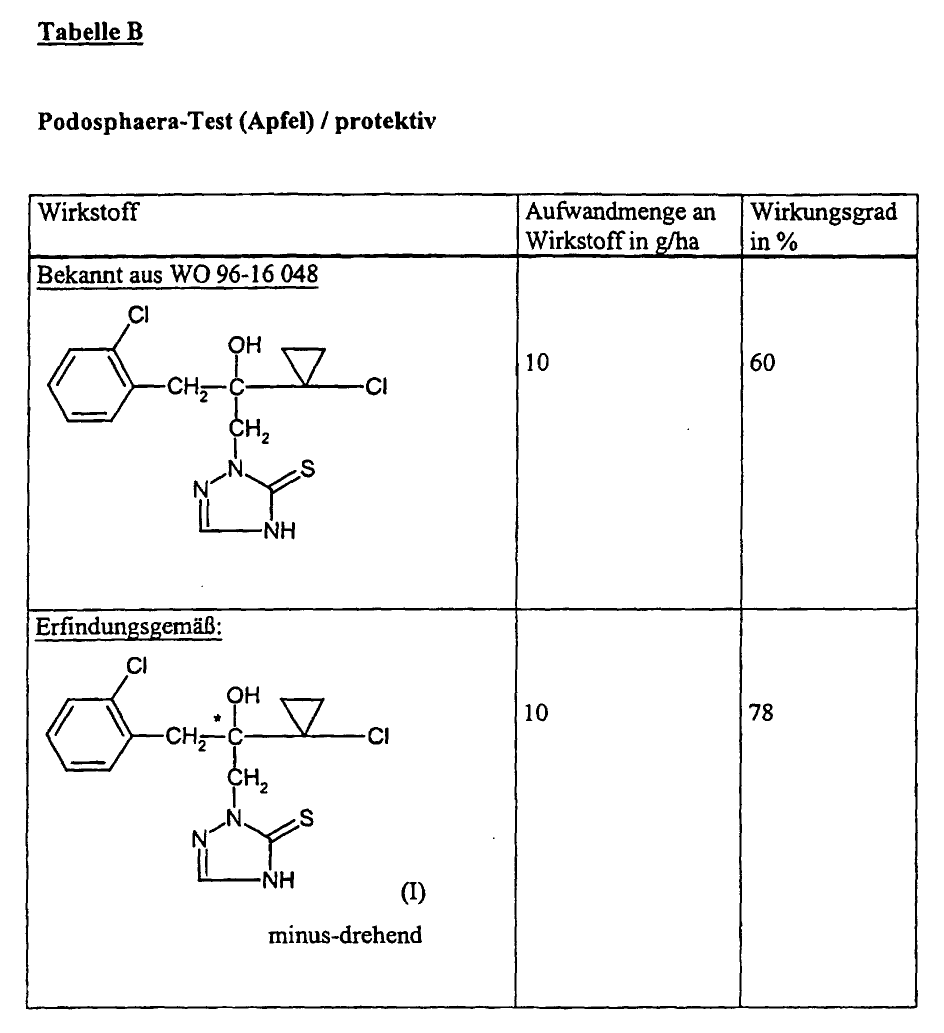

### Beispiel C

### Uncinula-Test (Rebe) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Uncinula necator* inokuliert. Die Pflanzen werden dann im Gewächshaus bei ca. 23°C und einer relativen Luftfeuchtigkeit von ca. 70 % aufgestellt.

14 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

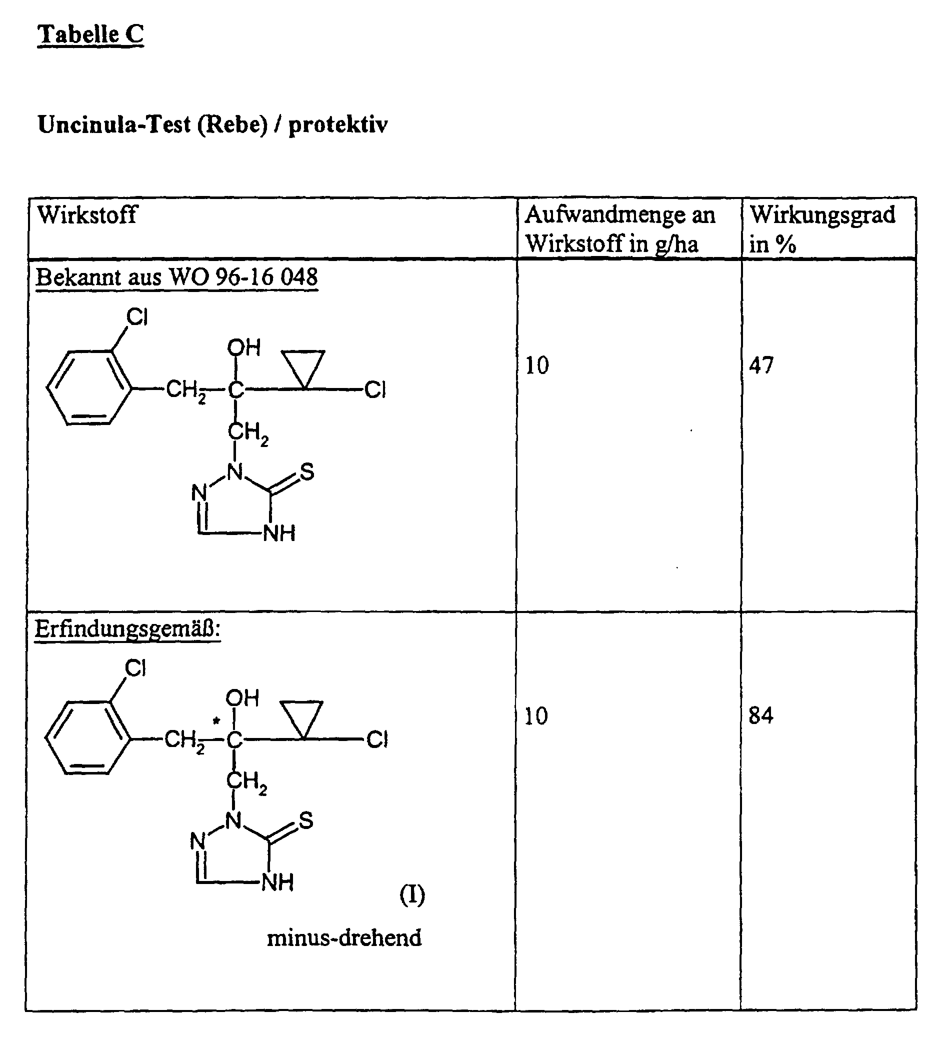

### Beispiel D

### Venturia-Test (Apfel) / kurativ

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wässrigen Konidiensuspension des Apfelschorferregers *Venturia inaequalis* inokuliert. Die Pflanzen verbleiben 1 Tag bei ca. 20°C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung in der angegebenen Aufwandmenge besprüht.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

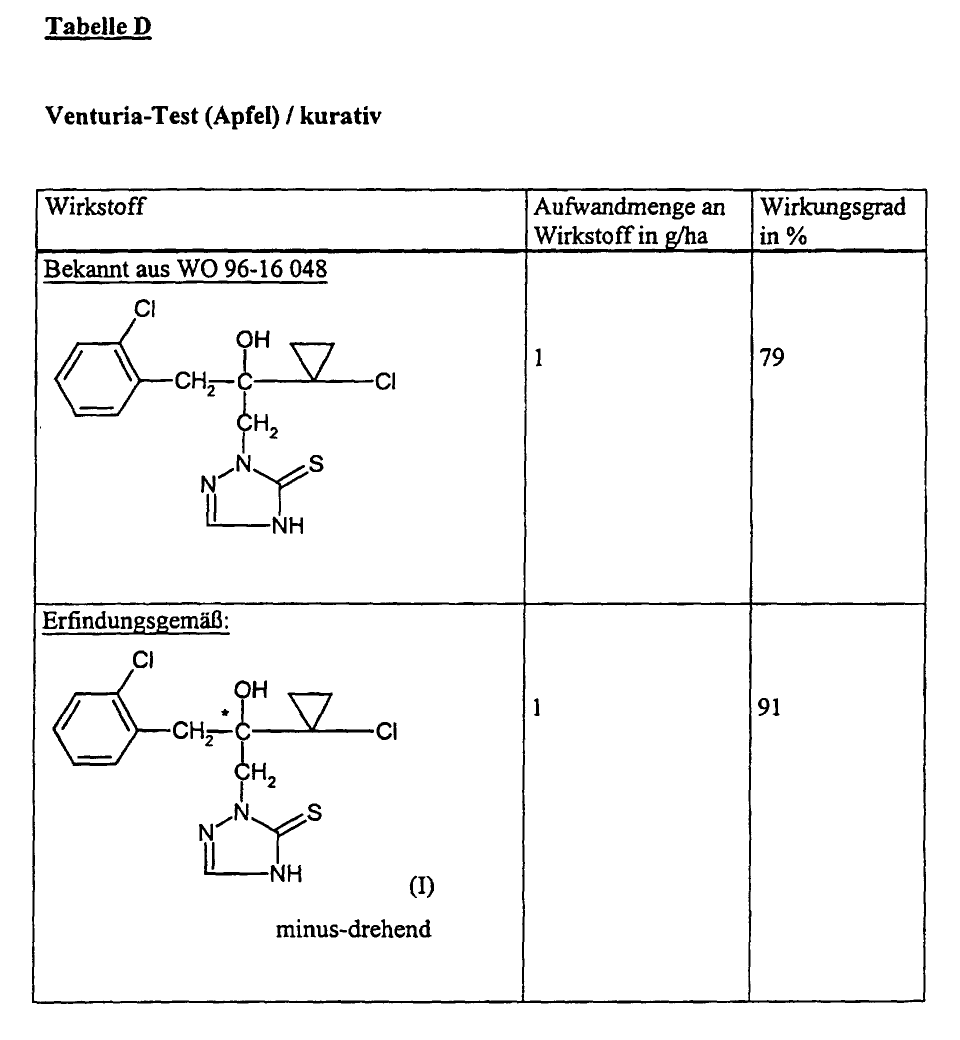

### Beispiel E

### Pyricularia-Test (Reis) / protektiv

| | | |
|---|---|---|
| Lösungsmittel | 2,5 | Gewichtsteile Aceton |
| Emulgator | 0,06 | Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wässrigen Sporensuspension von *Pyricularia oryzae* inkokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

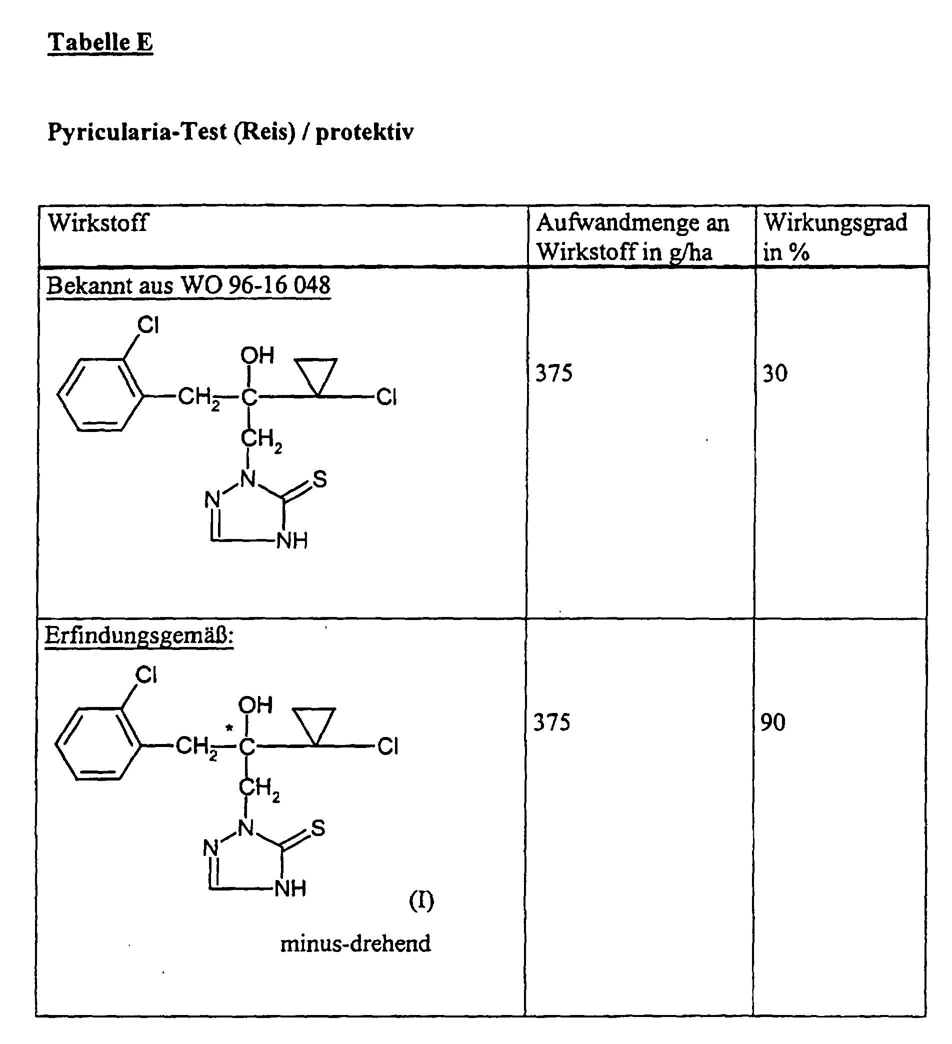

## Patentansprüche

1. (-)-Enantiomeres des 2-[2-(1-Chlor-cyclopropyl)-3-(2-chlor-phenyl)-2-hydroxypropyl]-2,4-dihydro-[1,2,4]-triazol-3-thions der Formel

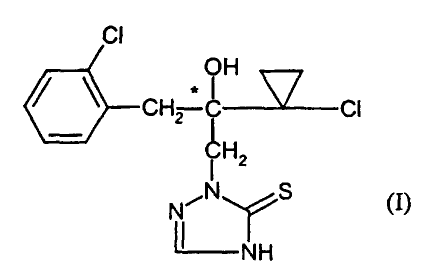

2. Verfahren zur Herstellung des (-)-Enantiomeren des Wirkstoffes der Formel (I) gemäß Anspruch 1, **dadurch gekennzeichnet, dass** man
a) racemisches 2-[2-(1-Chlor-cyclopropyl)-3-(2-chlor-phenyl)-2-hydroxypropyl]-2,4-dihydro-[1,2,4]-triazol-3-thion der Formel 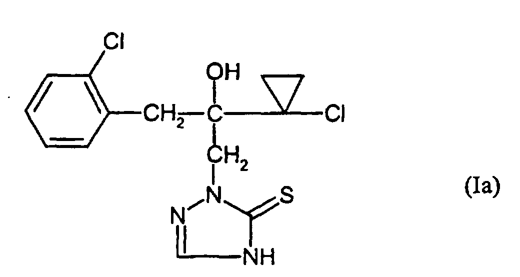 an einer chiralen stationären Kieselgelphase basierend auf dem optisch aktiven Monomer N-Methacryloyl-L-leucin-3-(2,4-dimethylpentyl)-amid mit Ethylacetat als Elutionsmittel bei Temperaturen zwischen 20°C und 25°C chromatographiert,
b) das Eluat unter vermindertem Druck einengt und
c) das dabei anfallende Produkt aus Toluol umkristallisiert.

3. Mikrobizide Mittel, **gekennzeichnet durch** einen Gehalt an dem (-)-Enantiomeren des Wirkstoffes der Formel (I) gemäß Anspruch 1 neben Streckmitteln und/oder oberflächenaktiven Stoffen.

4. Verwendung des (-)-Enantiomeren des Wirkstoffes der Formel (I) gemäß Anspruch 1 zur Bekämpfung von unerwünschten Mikroorganismen im Pflanzenschutz und im Materialschutz.

5. Verfahren zur Bekämpfung von unerwünschten Mikroorganismen im Pflanzenschutz und im Materialschutz, **dadurch gekennzeichnet, dass** man das (-)-Enantiomere des Wirkstoffes der Formel (I) gemäß Anspruch 1 auf die Mikroorganismen und/oder deren Lebensraum ausbringt.

6. Verfahren zur Herstellung von mikrobiziden Mitteln, **dadurch gekennzeichnet, dass** man das (-)-Enantiomere des Wirkstoffes der Formel (I) gemäß Anspruch 1 mit Streckmitteln und/oder oberflächenaktiven Stoffen vermischt.

## Claims

1. (-)-Enantiomer of 2-[2-(1-chloro-cyclopropyl)-3-(2-chloro-phenyl)-2-hydroxypropyl]-2,4-dihydro-[1,2,4]-triazole-3-thione of the formula

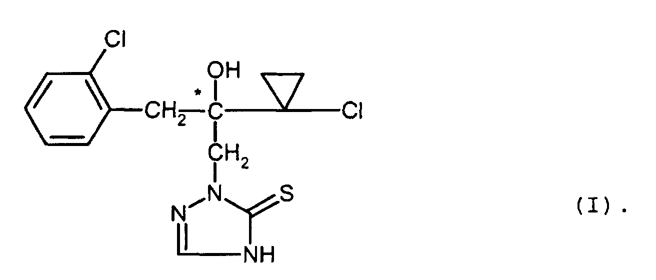

2. Process for preparing the (-)-enantiomer of the active compound of the formula (I) according to Claim 1, **characterized in that**
a) racemic 2-[2-(1-chloro-cyclopropyl)-3-(2-chloro-phenyl)-2-hydroxypropyl]-2,4-dihydro-[1,2,4]-triazole-3-thione of the formula 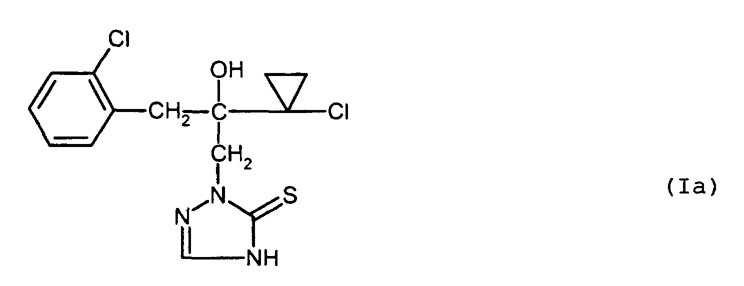 is chromatographed on a chiral stationary silica gel phase based on the optically active monomer N-methacryloyl-L-leucine-3-(2,4-dimethylpentyl)-amide using ethyl acetate as mobile phase at temperatures between 20°C and 25°C,
b) the eluate is concentrated under reduced pressure and
c) the resulting product is recrystallized from toluene.

3. Microbicidal composition, **characterized in that** it comprises the (-)-enantiomer of the active compound of the formula (I) according to Claim 1, in addition to extenders and/or surfactants.

4. Use of the (-)-enantiomer of the active compound of the formula (I) according to Claim 1 for controlling undesirable microorganisms in crop protection and the protection of materials.

5. Method for controlling undesirable microorganisms in crop protection and the protection of materials, **characterized in that** the (-)-enantiomer of the active compound of the formula (I) according to Claim 1 is applied to the microorganisms and/or their habitats.

6. Process for preparing microbicidal compositions, **characterized in that** the (-)-enantiomer of the active compound of the formula (I) according to Claim 1 is mixed with extenders and/or surfactants.

## Revendications

1. (-)-énantiomère de la 2-[2-(1-chlorocyclopropyl)-3-(2-chlorophényl)-2-hydroxypropyl]-2,4-dihydro-[1,2,4]-triazole-3-thione de formule générale

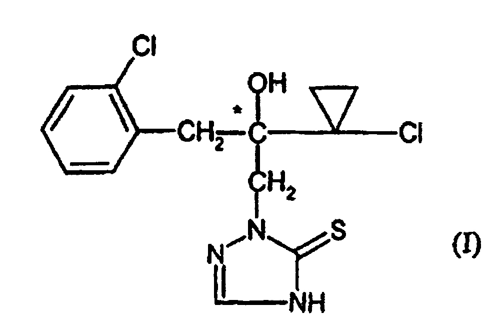

2. Procédé de production du (-)-énantiomère de la substance active de formule (I) suivant la revendication 1, **caractérisé en ce que**
a) on chromatographie la 2-[2-(1-chlorocyclopropyl)-3-(2-chlorophényl)-2-hydroxypropyl]-2,4-dihydro-[1,2,4]-triazole-3-thione racémique de formule 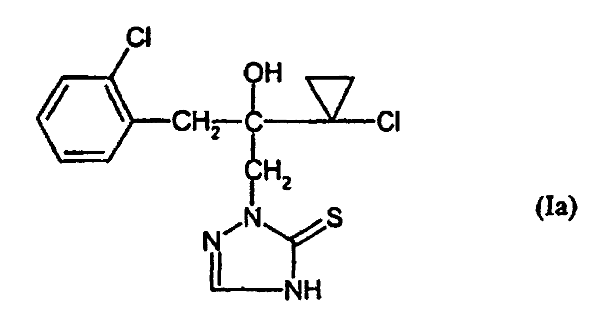 sur une phase chirale stationnaire de gel de silice à base du monomère optiquement actif 3-(2,4-diméthylpentyl)-amide de N-méthacryloyl-L-leucine avec l'acétate d'éthyle comme éluant à des températures comprises entre 20°C et 25°C,
b) on concentre l'éluat sous pression réduite et
c) on fait recristalliser dans du toluène le produit ainsi obtenu.

3. Compositions microbicides, **caractérisées par** une teneur en le (-)-énantiomère de la substance active de formule (I) suivant la revendication 1, à côté de diluants et/ou d'agents tensioactifs.

4. Utilisation du (-)-énantiomère de la substance active de formule (I) suivant la revendication 1 pour la lutte contre des micro-organismes indésirables dans la protection des plantes et dans la protection des matériaux.

5. Procédé pour lutter contre des micro-organismes indésirables dans la protection des plantes et dans la protection des matériaux, **caractérisé en ce qu'**on épand le (-)-énantiomère de la substance active de formule (I) suivant la revendication 1 sur les micro-organismes et/ou sur leur milieu.

6. Procédé de préparation de compositions microbicides, **caractérisé en ce qu'**on mélange le (-)-énantiomère de la substance active de formule (I) suivant la revendication 1 avec des diluants et/ou des agents tensioactifs.
